Europäisches Patentamt

European Patent Office

Office européen des brevets

(19)

(11) **EP 1 302 765 A2**

(12) **EUROPÄISCHE PATENTANMELDUNG**

(43) Veröffentlichungstag:
**16.04.2003   Patentblatt 2003/16**

(51) Int Cl.[7]: **G01N 17/00**

(21) Anmeldenummer: **02021505.9**

(22) Anmeldetag: **26.09.2002**

(84) Benannte Vertragsstaaten:
**AT BE BG CH CY CZ DE DK EE ES FI FR GB GR
IE IT LI LU MC NL PT SE SK TR**
Benannte Erstreckungsstaaten:
**AL LT LV MK RO SI**

(30) Priorität: **09.10.2001   DE 10149613**

(71) Anmelder: **Goldschmidt AG**
**45127 Essen (DE)**

(72) Erfinder:
• **Klocker, Otto**
  **45277 Essen (DE)**
• **Kleinsteinberg, Frank**
  **45481 Mülheim (DE)**

(54) **Verfahren zur Überprüfung der Verschmutzungsneigung von Anstrichen**

(57)    Verfahren zur Kontaminierung von Lacken, Farben und Beschichtungen bei dem eine wässrige Schmutzdispersion/-lösung, enthaltend im Wesentlichen anorganische Partikel, mittels einer Förderpumpe (1) aus einem Vorratsbehälter (2) über eine mit Düsen (4) ausgerüstete Rohrleitung (3) auf darunter mit einer Neigung von 45° zur Düsenöffnung befestigte Probekörper (4) gesprüht, die ablaufende Testflüssigkeit in einer Wanne (5) aufgefangen und zurück in den Vorratsbehälter geleitet wird.

**Beschreibung**

Gegenstand der Erfindung ist ein Verfahren zur schnellen und zuverlässigen Überprüfung der Verschmutzungsneigung bzw. des Selbstreinigungsvermögens von Anstrichen.

Oberflächen, insbesondere solche die permanent der Außenatmosphäre ausgesetzt sind, aber auch mit mechanischen oder chemischen Substanzen kontaminierte Innenatmosphären in Handwerksbetrieben sowie der Industrie, werden mit den verschiedenartigen Schmutzarten verunreinigt.

Zur Reinigung dieser Oberflächen ist häufig ein großer Aufwand erforderlich. Die Reinigungsmittelhersteller bieten eine Vielzahl von chemischen Mitteln an, die manuell oder maschinell appliziert werden und teilweise gute Ergebnisse erzielen.

Problematisch wird die Reinigung allerdings an unzugänglichen Stellen wie beispielsweise unregelmäßigen Gebäudestrukturen und Industrieanlagen. Aber insbesondere auch aus ökonomischen und ökologischen Gründen werden in der Praxis heute Beschichtungen und Farben gefordert, die mit schmutzabweisenden Verbindungen ausgestattet sind und daher eine Verschmutzung verhindern oder, wenn dies nicht in ausreichendem Maße möglich ist, zumindest vermindern und die gleichzeitig eine Reinigung ohne chemische Mittel, d.h. möglichst mit (Regen-)Wasser gestatten.

Eine zügige Entwicklung solcher Beschichtungen und Farben setzt aber ein Verfahren voraus, das den Praxisbedingungen möglichst nahe kommt und mit dem schnelle und zuverlässige Ergebnisse reproduzierbar erzielt werden können.

Zur Überprüfung der Verschmutzungsneigung von Lacken und Farben für Außenanstriche, insbesondere von Fassadenfarben, stand bisher als alleinige aussagefähige Messmethode die Freibewitterung zur Verfügung.

Diese Messmethode ist zwar praxisgerecht, hat jedoch den Nachteil, dass sie sehr zeitaufwendig ist. Erst nach einem Zeitraum von einigen Monaten, manchmal auch erst nach einigen Jahren sind Unterschiede im Verschmutzungsverhalten der Probepanels erkennbar.

In Laboratorien wurde daher versucht, die Verschmutzung von Farben mit sogenannten Handversuchen nachzustellen. Dabei wurden die Probepanels mit Staub bestreut oder mit mehr oder weniger praxisnahen Schmutzlösungen übergossen. Die Ergebnisse waren sehr personenabhängig, sehr zeitaufwendig und nur unzulänglich reproduzierbar.

Aus Process in Organic Coatings, Vol 27 (1966) Seite 173-180, Toshikazu Nakaya, Development of Staining Preventive Coating for Architekture, ist ein Verfahren bekannt, gemäß dem standardisierte Schmutzzusammensetzungen maschinell auf die zu überprüfenden Lackoberflächen aufgebracht werden (Abb. 1)

Abb. 1

Trockene
Luft

(d)

Rotation

(a)

(b)

(c)

Die zu testende Farbe wird hierbei auf Glasplatten (a) appliziert, die dann auf einem drehbaren Gestell fixiert werden. Über ihnen befindet sich ein Schöpfbecher (b) . Bei Drehung des Gestells wird der Schöpfbecher mit der wässrigen Lösung/Dispersion des Schmutzes gefüllt (c) und bei weiterer Drehung über die Probepanels entleert (d). Danach erfolgt eine Trocknung der Platten mit heißer Luft.

Aufgrund der starken Sedimentationsneigung der Dispersion verringert sich aber die Konzentration der Schmutzteilchen in der oberen Schicht sehr schnell, so dass nicht über die gesamte Testdauer eine repräsentative Menge der Schmutzpartikel vom Schöpfbecher erfasst werden kann.

Bei einer konstanten Verwirbelung des Sediments durch einen Bodenrührer wurde gefunden, dass die Schmutzteilchen zu Partikeln koagulieren, die zum einen in dieser Größe in der Praxis nicht auftreten und zum anderen im Test leichter entfernbar sind und somit als Ergebnis eine geringere Verschmutzungsneigung der Lacke vortäuschen als tatsächlich vorhanden.

Aufgabe der vorliegenden Erfindung war es daher, diese Nachteile der bekannten Testmethoden zu vermeiden und ein Verfahren zur schnellen und zuverlässigen Überprüfung der Verschmutzungsneigung bzw. des Selbstreinigungsvermögens von Anstrichen zur Verfügung zu stellen.

Diese Aufgabe wird gelöst durch ein Verfahren zur Kontaminierung von Lacken, Farben und Beschichtungen bei dem eine wässrige Schmutzdispersion/-lösung, enthaltend im Wesentlichen anorganische Partikel, mittels einer Förderpumpe (1) aus einem Vorratsbehälter (2) über eine mit Düsen (4) ausgerüstete Rohrleitung (3) auf darunter mit einer Neigung von 45° zur Düsenöffnung befestigte Probekörper (5) gesprüht, die ablaufende Testflüssigkeit in einer Wanne (6) aufgefangen und zurück in den Vorratsbehälter geleitet wird (Abb. 2).

Abbildung 2

von oben:

Während der gesamten Testdauer ist die Konzentration und die Größenverteilung der Teilchen in der Dispersion weitgehend unverändert, so dass mit dem erfindungsgemäßen Verfahren innerhalb von 3 Stunden eine signifikante, reproduzierbare Aussage über die Verschmutzungsneigung bzw. das Selbstreinigungsvermögen von Lacken oder Farben gegeben werden kann.

Durch Zusatz von handelsüblichen Dispergiermitteln und gegebenenfalls auch weiteren organischen Substanzen kann die Verteilung der Schmutzpartikel und der Erhalt der Partikelgröße in dem wässrigen Medium weiter optimiert werden.

Die organische Substanz und das Dispergiermittel dienen der besseren Verteilung des Schmutzes im Wasser und damit der Effektivität des Schmutztests. Wird der Schmutztest ohne diese Zusätze durchgeführt, ändert sich nur die Stärke der Verschmutzung, der Trend der Verschmutzung ändert sich nicht. Geringfügige Unterschiede in der Anschmutzneigung zweier Farben sind somit besser zu erkennen.

Als organische Substanzen können Lösungsmittel für die als weitere Schmutzbestandteile zusetzbaren Teere mitverwendet werden wie beispielsweise höhere aliphatische, cycloaliphatische und/oder aromatische Kohlenwasserstoffe, protische und aprotische polare Lösungsmittel wie ein oder mehrwertige Alkohole, Polyoxyalkylenglykole, Ether, Ketone.

Wird die Herstellung der Schmutzlösung noch zusätzlich bei Temperaturen von 60 °C bis 80 °C durchgeführt, so ist ein Absetzen des Schmutzes auch nach ca. 8 Stunden nicht gegeben.

Die Zusätze sollten aber in sehr geringen Konzentration eingesetzt werden, da sonst die Testdispersion mit einer Freibewitterung nicht mehr vergleichbar ist.

Zur Durchführung des erfindungsgemäßen Verfahrens wurde der in der oben zitierten Literaturstelle beschriebene Normstaub Nummer 8 verwendet und zusätzlich Ruß und Teer.

Rezeptur des Schmutzes:

| Komponente | Einwaage |
|---|---|
| Normstaub Nr. 8 | 70,0 g |
| FW 200 (Degussa) | 17,0 g |
| Special Pitch No. 5 (Fa. Worlee) | 13,0 g |

Beschreibung der Komponenten:

- FW 200 der Firma Degussa
  Gasruß (feinteilig)

- Special Pitch No. 5
  Beim Special Pitch No. 5 handelt es sich um ein komplexes Gemisch aus aromatischen Kohlenwasserstoffen in Xylol, der im Wesentlichen die folgenden Bestandteile enthält:

| Xylol | 12,5-15 % |
|---|---|
| Benzopyren | $\leq$ 0,7 % |
| Benzoanthracen | $\leq$ 1 % |
| Benzofluoranthen | $\leq$ 1,4 % |
| Dibenzanthracen | $\leq$ 0,1 % |
| Naphthalin | $\leq$ 2,6 % |

- Normstaub Nr. 8
  Der Normstaub Nr. 8 ist nach umfangreicher Analytik in Japan entwickelt worden. Er enthält Substanzen, die in der Form im Staub der Stadt Tokyo nachgewiesen wurden.

Inhaltsstoffe:

| $SiO_2$ | 34-40 % |
|---|---|
| $Fe_2O_3$ | 17-23 % |
| $Al_2O_3$ | 26-32 % |
| $CaO$ | $\leq$ 3 % |
| $MgO$ | 3-7 % |
| $TiO_2$ | $\leq$ 4 % |

Korngrößenverteilung:

| Größe in µm | Gehalt in % |
|---|---|
| 5 | 61 ± 5 |
| 10 | 43 ± 3 |
| 20 | 27 ± 3 |
| 30 | 15 ± 3 |
| 40 | 9 ± 3 |
| 75 | 3 |

Herstellung des Schmutzes

1. Die Komponenten der Rezeptur werden in eine 250-ml-Pulverglasflasche eingewogen.
2. Es werden ca. 100 g Glasperlen (Ø ca. 0,5 mm) zugegeben.
3. Die Flasche wird gut verschlossen und 1 Stunde im Scandex-Shaker geschüttelt.
4. Die Glasperlen werden abgesiebt.
5. Dann wird das Schmutzpulver mit Mörser und Pistill nochmals homogenisiert und danach wird das Pulver mit einem feinmaschigen Sieb ( 0,063 mm) abgesiebt.

Herstellung der Schmutzlösung/-dispersion

| Rezeptur: | |
|---|---|
| Schmutzpulver, s.o. | 5,00 g |
| Organische Substanz (1,0 g Dodecan 4,0 g Butylgylkol) | 5,00 g |
| Dispergiermittel z.B. Surfynol 141 oder TEGO Dispers 760 | 1,00 g |
| Entschäumer z.B. TEGO Foamex 3062 | 1,00 g |
| Wasser (7°dH) | 988,00 g |
| Zusatzwasser (7°dH) | 4000,00 g |

988 g Wasser werden in ein 5-l-Gefäß eingewogen und auf eine Temperatur von 75 °C erwärmt. Danach werden organische Substanz, Dispergiermittel und Entschäumer unter Rühren zugegeben. Das Schmutzpulver wird dann langsam in das warme Wasser eingestreut. Das Pulver wird dann mittels einem Ultraturrax bei 10.000 rpm 15 min dispergiert. Das Zusatzwasser wird dann portionsweise unter Rühren (Utraturrax: ca. 4000 rpm) so dazu gegeben, dass die Temperatur der Mischung am Ende der Wasserzugabe bei < 30°C war (eventuell zusätzliche Kühlung erforderlich).

Diese Schmutzlösung wird vor jedem Verschmutzungstest frisch hergestellt.

Durchführung des Schmutztests

Probenvorbereitung

Die zu prüfende Farbe wird mit einem Kastenrakel (Schichtdicke 100 µm nass) auf eine saubere, fettfreie Glasplatte (Größe 100 x 150 mm) appliziert und 24 h bei 50 °C im Umluftofen getrocknet. Nach weiteren zwei Stunden Konditionierung bei Raumtemperatur wird der L*-Wert (Reflektionswert) der Beschichtung ($L^*_{vor}$) vor weißem Hintergrund (L* = 95; a* = 0,1; b* = 0,5) gemessen und notiert.

Vorbereitung des Gerätes

Die Schmutzlösung wird vor jedem Schmutztest hergestellt und in den Vorratsbehälter des Gerätes gefüllt.

Um das Gemisch zu homogenisieren, wird die Pumpe eingeschaltet und die Lösung drei Mal umgepumpt.

Ablauf des Verfahrens

- Das Gerät wird mit den Probepanels ( 2 Reihen mit jeweils 3 Platten) bestückt.
- Der Zähler des Geräts wird auf Null gestellt und die Pumpe eingeschaltet.
- Das Gerät pumpt nun 30 mal je 250 ml Schmutzlösung innerhalb von fünf Sekunden über jede Glasplatte. Dazwischen ist jedes Mal eine Pause von fünf Sekunden. Ein Zyklus dauert also ca. fünf Minuten.
- Das Gerät unterbricht automatisch nach 30 Pumpvorgängen.
- Die Platten werden aus dem Gerät entfernt und sechs Minuten bei 50°C im Umluftofen getrocknet. Das Gerät ruht in dieser Zeit. Ein Umpumpen der Schmutzlösung, um Absetzen zu vermeiden ist nicht nötig.
- Anschließend werden die Platten wieder im Gerät 30 mal mit Schmutzlösung besprüht. Bei der Platzierung der Platten im Gerät ist ein bestimmter Platzwechsel einzuhalten, der noch näher beschrieben wird.
- Dieser Vorgang wird 10 mal wiederholt.

Während eines Schmutztests wird eine Probe also 10 x 30 = 300 mal mit 250 ml Schmutzlösung übergossen.

Auswertung

Nach abschließender Trocknung der Platten bei 50° C im Umluftofen wird wiederum der L*-Wert der Beschichtung (L*$_{nach}$) vor weißem Hintergrund mit dem Spektrometer SP 68 der Firma X-Rite gemessen und notiert.
Der Verschmutzungsgrad (Vg) der Beschichtung in Prozent wird dann nach folgender Gleichung berechnet:

$$Vg = \frac{L*_{vor} - L*_{nach}}{L*_{vor}} \cdot 100\% = \left(1 - \frac{L*_{nach}}{L*_{vor}}\right) \cdot 100\%$$

Aus der Gleichung ist ersichtlich, dass es sich bei dem Verschmutzungsgrad Vg um die relative Helligkeitsabnahme der Beschichtung durch die Verschmutzung handelt.
Würden sehr unterschiedlich helle Beschichtungen verglichen, wäre es sinnvoller, die absolute Abnahme des L*-Wertes oder den ∆E-Wert zu vergleichen.

Reproduzierbarkeit des Tests

Um die Reproduzierbarkeit des Schmutztests zu ermitteln, wurden verschiedene Variationen der Rezeptur TS (Micro-Siliconharzfarbe) auf Basis verschiedener Dispersionen mehrmals dem Test unterzogen.
Wurde auf gezielte Platzierung der Platten im Verschmutzungsgerät verzichtet, ergaben sich nach drei Testdurchläufen folgende Ergebnisse:
Ergebnisse dreier Testdurchläufe bei zufälliger Panelplatzierung

| Rezeptur | TS-1 | TS-2 | TS-3 | TS-4 | TS-5 | TS-6 |
|---|---|---|---|---|---|---|
| **Vg Test1** | 62 % | 54,8 % | 63,5 % | 45,2 % | 72 % | 50 % |
| **Vg Test2** | 50,8 % | 51,9 % | 59,5 % | 43,9 % | 66,1 % | 40,8 % |
| **Vg Test3** | 51,9 % | 55,9 % | 55,6 % | 47,2 % | 65 % | 44,5 % |
| **Mittelwert** | 54,9 % | 54,2 % | 59,5 % | 45,4 % | 67,7 % | 45,1 % |
| **Standard-abweichung** | 5,0 | 1,7 | 3,2 | 1,4 | 3,1 | 3,8 |

Die große Abweichung der Ergebnisse ist auf die unterschiedlichen Drücke an den Düsen des Verschmutzungsgeräts zurückzuführen. Zur Kompensierung dieses Fehlers ist daher darauf zu achten, dass jede Platte möglichst an jedem Platz im Gerät verschmutzt wurde.

Erfindungsgemäß wird die Platzierung der Platten nach folgendem Schema durchgeführt:

Die Platte, die beim ersten Zyklus auf Platz 1 verschmutzt wurde, wird beim zweiten Zyklus auf Platz 2 verschmutzt. Die Platte von Platz 2 wandert auf Platz 3. Die Platte von Platz 3 auf Platz 1. Mit Platz 4-6 wird entsprechend verfahren. Bei jedem weiteren Zyklus wird ebenso verfahren. Nach dem fünften Zyklus wird zusätzlich die Seite gewechselt, d.h. Platz 1-3 wechselt mit Platz 4-6.

Durch diese gezielte Platzierung der Platten wird gewährleistet, dass auch mögliche zufällige Unregelmäßigkeiten bei der Durchführung des Verfahrens kompensiert und jede Platte gleich verschmutzt wird.

Wurde der Schmutztest nach diesem gezielten Schema ausgeführt, ergaben sich folgende Ergebnisse:

| Rezeptur | TS-1 | TS-2 | TS-3 | TS-4 | TS-5 | TS-6 |
|---|---|---|---|---|---|---|
| **Vg Test4** | 61,1% | 57,3% | 59,1% | 46,2% | 69,2% | 45,5% |
| **Vg Test5** | 59,1% | 55,5% | 60,3% | 44,3% | 69,8% | 45,8% |
| **Vg Test6** | 57,1% | 58,1% | 56,3% | 44,5% | 67,7% | 45,3% |
| **Mittelwert** | 59,1% | 57,0% | 58,6% | 45,0% | 68,9% | 45,5% |
| **Standard-abweichung** | 1,6 | 1,1 | 1,7 | 0,9 | 0,9 | 0,2 |

Es ist deutlich erkennbar, dass der Test nun eine zufriedenstellende Reproduzierbarkeit besitzt.

Korrelation zur Freibewitterung

Es wurden drei verschiedene Farben jeweils dem erfindungsgemäßen Verfahren und einer Freibewitterung unterzogen:

TEGOsan -         selbstformulierte Fassadenfarbe auf Basis einer Styrol/Acrylatdispersion und eines Aminsiloxans

SHF -         selbstformulierte Fassadenfarbe auf Basis einer Styrol/Acrylatdispersion und eines Silikonharzes

Mikrosiliconharzfarbe -     handelsübliche Fassadenfarbe auf Basis einer Styrol/Acrylatdispersion und eines Silans

Die Proben wurden wie oben vorbereitet und 18 Monate bei einer Neigung von 45° Richtung Süden im Essener Stadtgebiet exponiert.

Eine Korrelation ist eindeutig erkennbar.

## Patentansprüche

1. Verfahren zur Kontaminierung von Lacken, Farben und Beschichtungen bei dem eine wässrige Schmutzdispersion/lösung, enthaltend im Wesentlichen anorganische Partikel, mittels einer Förderpumpe (1) aus einem Vorratsbehälter (2) über eine mit Düsen (4) ausgerüstete Rohrleitung (3) auf darunter mit einer Neigung von 45° zur Düsenöffnung befestigte Probekörper (4) gesprüht, die ablaufende Testflüssigkeit in einer Wanne (5) aufgefangen und zurück in den Vorratsbehälter geleitet wird.

2. Verfahren gemäß Anspruch 1, **dadurch gekennzeichnet, dass** zur weiteren Stabilisierung der Dispersion Dispergierhilfsmittel und gegebenenfalls weitere organische Substanzen mitverwendet werden.

3. Verfahren gemäß Anspruch 1, **dadurch gekennzeichnet, dass** das Verfahren bei Temperaturen von 60°C bis 80°C durchgeführt wird.